# EUROPEAN PATENT SPECIFICATION

(11) **EP 1 841 317 B1**
(45) Date of publication and mention of the grant of the patent: **11.03.2009**
(21) Application number: 04803436.7
(22) Date of filing: 02.12.2004
(51) Int. Cl.: A01N 37/52

(54) **THE USE OF N-ARYLHYDRAZINE DERIVATIVES FOR COMBATING PESTS IN AND ON ANIMALS**
VERWENDUNG VON N-ARYLHYDRAZINDERIVATEN FÜR DIE BEKÄMPFUNG VON SCHÄDLINGEN IN UND AN TIEREN
UTILISATION DE DERIVES DE N-ARYLHYDRAZINE POUR LA LUTTE CONTRE DES PARASITES CHEZ ET SUR DES ANIMAUX

(30) Priority: 04.12.2003 US 526609 P
(43) Date of publication of application: 10.10.2007
(73) Proprietor: BASF SE, 67056 Ludwigshafen (DE)
(72) Inventor: VON DEYN, Wolfgang, 67435 Neustadt (DE); OLOUMI-SADEGHI, Hassan, Raleigh, NC 27614 (US); KUHN, David G., Apex, NC 27502 (US); ARMES, Nigel, Raleigh NC 27613 (US); KORADIN, Christopher, 67067 Ludwigshafen (DE); ZELLER, Alissa, 68165 Mannheim (DE)
(86) International application number: PCT/EP2004/013685
(87) International publication number: WO 2005/053402

(56) References cited:
- EP-A- 0 604 798
- DATABASE CA [Online] CHEMICAL ABSTRACTS SERVICE, COLUMBUS, OHIO, US; KUHN, D. G. ET AL KUHN, D. G. ET AL: "Cycloalkyl-substituted amidrazones: a novel class of insect control agents Cycloalkyl-substituted amidrazones: a novel class of insect control agents" XP002330111 retrieved from STN Database accession no. 1998:294837 & ACS SYMPOSIUM SERIES , 686(SYNTHESIS AND CHEMISTRY OF AGROCHEMICALS V), 185-193 CODEN: ACSMC8; ISSN: 0097-6156 ACS SYMPOSIUM SERIES , 686(SYNTHESIS AND CHEMISTRY OF AGROCHEMICALS V), 185-193 CODEN: ACSMC8; ISSN: 0097-6156, 1998, cited in the application
- DATABASE CA [Online] CHEMICAL ABSTRACTS SERVICE, COLUMBUS, OHIO, US; FURCH, J. A. ET AL FURCH, J. A. ET AL: "Amidrazones: a new class of coleopteran insecticides Amidrazones: a new class of coleopteran insecticides" XP002330112 retrieved from STN Database accession no. 1998:294826 & ACS SYMPOSIUM SERIES , 686(SYNTHESIS AND CHEMISTRY OF AGROCHEMICALS V), 178-184 CODEN: ACSMC8; ISSN: 0097-6156 ACS SYMPOSIUM SERIES , 686(SYNTHESIS AND CHEMISTRY OF AGROCHEMICALS V), 178-184 CODEN: ACSMC8; ISSN: 0097-6156, 1998, cited in the application

## Description

The present invention relates to the use of compounds of the formula I 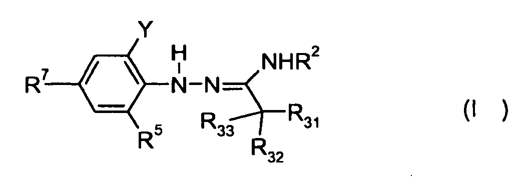 wherein
- R⁷: is chlorine or trifluoromethyl;
- R⁵ and Y: are each independently chlorine or bromine;
- R²: is C₁-C₆-alkyl, C₃-C₆-alkenyl, C₃-C₆-alkynyl, or
C₃-C₆-cycloalkyl which may be substituted with 1 to 3 halogen atoms, or
C₂-C₄-alkyl which is substituted by C₁-C₄-alkoxy;
- R³¹ and R³²: are C₁-C₆-alkyl or may be taken together to form C₃-C₆-cycloalkyl which may be unsubstituted or substituted by 1 to 3 halogen atoms;
- R³³: is hydrogen or C₁-C₆-alkyl,
or the enantiomers or veterinarily acceptable salts thereof,
for combating parasites in and on animals.

It is generally a goal of agronomists and veterinarians to possess sufficient means to control parasites, when they attempt to invade or attack animals.

It is an object of the present invention to provide new methods to control parasites in and on animals. Another object of the invention is to provide safer pesticides for animals. Another object of the invention is to provide pesticides for animals that may be used in lower doses than existing pesticides. Another object of the invention is to provide pesticides for animals which provide a long residual control of the parasites.

These objects are met in whole or in part by the present invention.

The invention also relates to compositions containing a parasiticidally effective amount of compounds of formula I and an acceptable carrier, for combating parasites in and on animals.

The present invention also provides a method for treating, controlling, preventing and protecting animals against infestation and infection by parasites, which comprises orally, topically or parenterally administering or applying to the animals a parasiticidally effective amount of a compound of formula I or a composition comprising it.

The invention also provides a process for the preparation of a composition for treating, controlling, preventing or protecting animals against infestation or infection by parasites which comprises combining a parasiticidally effective amount of a compound of formula I or a composition comprising it with an acceptable carrier.

The insecticidal and acaricidal activity in crop protection of some of the compounds of formula I has been described in EP-A 604 798, and also in J. A Furch et al., "Amidrazones: A New Class of Coleopteran Insecticides", ACS Symposium Series 686, Am. Chem. Soc., 1998, Chapter 18, p. 178 ff, and also in D. G. Kuhn et al., "Cycloalkyl-substituted Amidrazones: A Novel Class of Insect Control Agents", ACS Symposium Series 686, Am. Chem. Soc., 1998, Chapter 19, p. 185 ff.

Activity of compounds against agricultural pests does not suggest their suitability for control of endo- and ectoparasites in and on animals which requires, for example, low, non-emetic dosages in the case of oral application, metabolic compatibility with the animal, low toxicity, and a safe handling.

Surprisingly it has now been found that compounds of formula I are suitable for combating endo- and ectoparasites in and on animals.

The compounds of formula I can be prepared according to preparation methods described or referenced in EP-A 604 798 or modifications thereof.

In the definition of formula I shown above, the substituents have the following meanings:

"Halogen" will be taken to mean fluoro, chloro, bromo and iodo.

The term "alkyl" as used herein refers to a branched or unbranched saturated hydrocarbon group having 1 to 10 carbon atoms, especially C₁-C₆-alkyl such as methyl, ethyl, propyl, 1-methylethyl, butyl, 1-methylpropyl, 2-methylpropyl, 1,1-dimethylethyl, pentyl, 1-methylbutyl, 2-methylbutyl, 3-methylbutyl, 2,2-dimethylpropyl, 1-ethylpropyl, hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl and 1-ethyl-2-methylpropyl.

The term "haloalkyl" as used herein refers to a straight-chain or branched alkyl groups having 1 to 10 carbon atoms (as mentioned above), where some or all of the hydrogen atoms in these groups may be replaced by halogen atoms as mentioned above, for example C₁-C₂-haloalkyl, such as chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl and pentafluoroethyl;

"Alkoxy" refers to straight-chain or branched alkyl group having 1 to 4 or 6 carbon atoms (as mentioned above) bonded through an oxygen linkage, at any bond in the alkyl group. Examples include methoxy, ethoxy, propoxy, and isopropoxy.

The term "alkenyl" as used herein intends a branched or unbranched unsaturated hydrocarbon group having 3 to 10 carbon atoms and a double bond in any position, such as C₃-C₆ alkenyl such as 1-propenyl, 2-propenyl, 1-methyl-ethenyl, 1-butenyl, 2-butenyl, 3-butenyl, 1-methyl-1-propenyl, 2-methyl-1-propenyl, 1-methyl-2-propenyl, 2-methyl-2-propenyl; 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl and 1-ethyl-2-methyl-2-propenyl;

"Cycloalkyl" refers to a monocyclic 3- to 6-, 8-, 10- or 12-membered saturated carbon atom rings, e.g. C₃-C₈-cycloalkyl such as cyclopropyl, cyclobutyl, cyclopentyl, cyclohexyl, cycloheptyl and cyclooctyl.

By the term "veterinarily acceptable salts" is meant salts the anions of which are known and accepted in the art for the formation of salts for veterinary use. Suitable acid addition salts, e.g. formed by compounds of formula I containing a basic nitrogen atom, e.g. an amino group, include salts with inorganic acids, for example hydrochlorids, sulphates, phosphates, and nitrates and salts of organic acids for example acetic acid, maleic acid, dimaleic acid, fumaric acid, difumaric acid, methane sulfenic acid, methane sulfonic acid, and succinic acid.

Preference is given to compounds of formula I wherein R⁷ is trifluoromethyl.

Preference is further given to compounds of formula I wherein Y and R⁵ are both chlorine.

Moreover, preferred are compounds of formula I wherein R² is C₁-C₆-alkyl, especially ethyl.

Preference is further given to compounds of formula I wherein R³¹ and R³² are both methyl.

Moreover, preferred are compounds of formula I wherein R³¹ and R³² form a cyclopropyl ring which is unsubstituted or substituted by 1 to 3 halogen atoms, especially chlorine and bromine.

Moreover, particularly preferred are compounds of formula I wherein R³¹ and R³² form a cyclopropyl ring which is substituted by 2 halogen atoms.

Moreover, particularly preferred are compounds of formula I wherein R³¹ and R³² form a cyclopropyl ring which is substituted by 2 chlorine atoms.

Particularly preferred are compounds of formula I wherein R³¹ and R³² form a 2,2-dichlorocyclopropyl ring.

Preference is further given to compounds of formula I wherein R³³ is C₁-C₆-alkyl, especially methyl.

Particularly preferred are compounds of formula I wherein R³¹, R³² and R³³ are all methyl.

Moreover, particularly preferred are compounds of formula I wherein R³¹, R³² and R³³ form a moiety 1-methyl-2,2-dichlorocyclopropyl.

Preference is further given to compounds of formula I wherein
R⁷ is trifluoromethyl;
Y and R⁵ are each independently chlorine or bromine;
R² is C₁-C₆-alkyl;
R³¹ and R³² are C₁-C₆-alkyl or may be taken together to form C₃-C₆-cycloalkyl which is substituted by 1 to 2 halogen atoms;
R³³ is C₁-C₆-alkyl;
or the enantiomers or veterinarily acceptable salts thereof.

Particular preference is given to N-ethyl-2,2-dimethylpropionamide-2-(2,6-dichloro-α,α,α-trifluoro-p-tolyl)hydrazone and N-Ethyl-2,2-dichloro-1-methylcyclopropane-carboxamide-2-(2,6-dichloro-α,α,α-trifluoro-p-tolyl)hydrazone.

Furthermore, particular preference with respect to the use in the present invention is given to the compound of formula I-1 (N-ethyl-2,2-dimethylpropionamide-2-(2,6-dichloro-α,α,α-trifluoro-p-tolyl)-hydrazone):

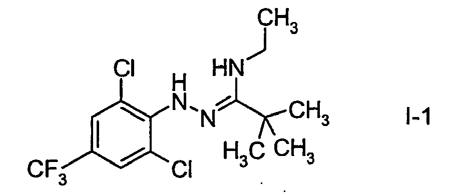

Moreover, particular preference with respect to the use in the present invention is given to the compound of formula I-2 (N-Ethyl-2,2-dichloro-1-methylcyclopropane-carboxamide-2-(2,6-dichloro-α,α,α -trifluoro-p-tolyl)hydrazone):

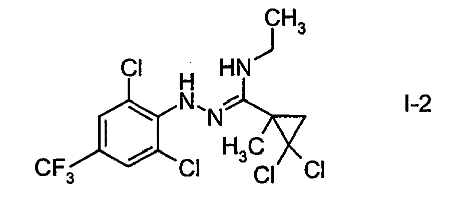

Compounds of formula I and compositions comprising them are preferably used for controlling and preventing infestations and infections animals including warm-blooded animals (including humans) and fish. They are for example suitable for controlling and preventing infestations and infections in mammals such as cattle, sheep, swine, camels, deer, horses, pigs, poultry, rabbits, goats, dogs and cats, water buffalo, donkeys, fallow deer and reindeer, and also in fur-bearing animals such as mink, chinchilla and raccoon, birds such as hens, geese, turkeys and ducks and fish such as fresh- and salt-water fish such as trout, carp and eels.

Compounds of formula I and compositions comprising them are preferably used for controlling and preventing infestations and infections in domestic animals, such as dogs or cats.

Infestations in warm-blooded animals and fish include, but are not limited to, lice, biting lice, ticks, nasal bots, keds, biting flies, muscoid flies, flies, myiasitic fly larvae, chiggers, gnats, mosquitoes and fleas.

The compounds of formula I and compositions comprising them are suitable for systemic and/or non-systemic control of ecto- and/or endoparasites. They are active against all or some stages of development.

The compounds of formula I are especially useful for combating ectoparasites.

The compounds of formula I are especially useful for combating parasites of the following orders and species, respectively:
fleas (Siphonaptera), e.g. *Ctenocephalides felis, Ctenocephalides canis, Xenopsylla cheopis, Pulex irritans, Tunga penetrans,* and *Nosopsyllus fasciatus,*
cockroaches (Blattaria - Blattodea), e.g. *Blattella germanica, Blattella asahinae, Periplaneta americana, Periplaneta japonica, Periplaneta brunnea, Periplaneta fuligginosa, Periplaneta australasiae,* and *Blatta orientalis,*
flies, mosquitoes (Diptera), e.g. *Aedes aegypti, Aedes albopictus, Aedes vexans, Anastrepha ludens, Anopheles maculipennis, Anopheles crucians, Anopheles albimanus, Anopheles gambiae, Anopheles freeborni, Anopheles leucosphyrus, Anopheles minimus, Anopheles quadrimaculatus, Calliphora vicina, Chrysomya bezziana, Chrysomya hominivorax, Chrysomya macellaria, Chrysops discalis, Chrysops silacea, Chrysops atlanticus, Cochliomyia hominivorax, Cordylobia anthropophaga, Culicoides furens, Culex pipiens, Culex nigripalpus, Culex quinquefasciatus, Culex tarsalis, Culiseta inornata, Culiseta melanura, Dermatobia hominis, Fannia canicularis, Gasterophilus intestinalis, Glossina morsitans, Glossina palpalis, Glossina fuscipes, Glossina tachinoides, Haematobia irritans, Haplodiplosis equestris, Hippelates spp., Hypoderma lineata, Leptoconops torrens, Lucilia caprina, Lucilia cuprina, Lucilia sericata, Lycoria pectoralis, Mansonia spp., Musca domestica, Muscina stabulans, Oestrus ovis, Phlebotomus argentipes, Psorophora columbiae, Psorophora discolor, Prosimulium mixtum, Sarcophaga haemorrhoidalis, Sarcophaga sp., Simulium vittatum, Stomoxys calcitrans, Tabanus bovinus, Tabanus atratus, Tabanus lineola,* and *Tabanus similis,*
lice (Phthiraptera), e.g. *Pediculus humanus capitis, Pediculus humanus corporis, Pthirus pubis, Haematopinus eurysternus, Haematopinus suis, Linognathus vituli, Bovicola bovis, Menopon gallinae, Menacanthus stramineus* and *Solenopotes capillatus.*
ticks and parasitic mites (Parasitiformes): ticks (Ixodida), e.g. *Ixodes scapularis, Ixodes holocyclus, Ixodes pacificus, Rhiphicephalus sanguineus, Dermacentor andersoni, Dermacentor variabilis, Amblyomma americanum, Ambryomma maculatum, Ornithodorus hermsi, Ornithodorus turicata* and parasitic mites (Mesostigmata), e.g. *Ornithonyssus bacoti* and *Dermanyssus gallinae,*
Actinedida (Prostigmata) und Acaridida (Astigmata) e.g. *Acarapis spp*., *Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp.,* Sarcoptes *spp., Notoedres spp.,Knemidocoptes spp., Cytodites spp.,* and *Laminosioptes spp,*
Bugs (Heteropterida): *Cimex lectularius, Cimex hemipterus, Reduvius senilis, Triatoma spp., Rhodnius ssp., Panstrongylus ssp.* and *Arilus critatus,*
Anoplurida, e.g. *Haematopinus spp., Linognathus spp., Pediculus spp., Phtirus spp.,* and *Solenopotes spp,*
Mallophagida (suborders Arnblycerina and Ischnocerina), e.g. *Trimenopon spp., Menopon spp., Trinoton spp., Bovicola spp., Werneckiella spp., Lepikentron spp., Trichodectes spp.,* and *Felicola spp,*
Roundworms Nematoda:
   Wipeworms and Trichinosis (Trichosyringida), e.g. Trichinellidae *(Trichinella spp.),* (Trichuridae) *Trichuris spp., Capillaria spp,*
   Rhabditida, e.g. *Rhabditis spp, Strongyloides spp., Helicephalobus spp,*
   Strongylida, e.g. *Strongylus spp., Ancylostoma spp., Necator americanus, Bunostomum spp. (Hookworm), Trichostrongylus spp., Haemonchus contortus., Ostertagia spp. , Cooperia spp., Nematodirus spp., Dictyocaulus spp., Cyathostoma spp., Oesophagostomum spp., Stephanurus dentatus, Ollulanus spp., Chabertia spp., Stephanurus dentatus , Syngamus trachea, Ancylostoma spp., Uncinaria spp., Globocephalus spp., Necator spp., Metastrongylus spp., Muellerius capillaris, Protostrongylus spp., Angiostrongylus spp., Parelaphostrongylus spp. Aleurostrongylus abstrusus,* and *Dioctophyma renale,*
   Intestinal roundworms (Ascaridida), e.g. *Ascaris lumbricoides, Ascaris suum, Ascaridia galli, Parascaris equorum, Enterobius vermicularis (Threadworm), Toxocara canis, Toxascaris leonine, Skrjabinema spp.,* and *Oxyuris equi,*
   Camallanida, e.g. *Dracunculus medinensis* (guinea worm)
   Spirurida, e.g. *Thelazia spp. Wuchereria spp., Brugia spp., Onchocerca spp., Dirofilari spp.a, Dipetalonema spp., Setaria spp., Elaeophora spp., Spirocerca lupi,* and *Habronema spp.,*
   Thorny headed worms (Acanthocephala), e.g. *Acanthocephalus spp., Macracanthorhynchus hirudinaceus* and *Oncicola spp,*
Planarians (Plathelminthes):
   Flukes (Trematoda), e.g. *Faciola spp., Fascioloides magna, Paragonimus spp., Dicrocoelium spp., Fasciolopsis buski, Clonorchis sinensis, Schistosoma spp., Trichobilharzia spp., Alaria alata, Paragonimus spp.,* and *Nanocyetes spp,*
   Cercomeromorpha, in particular Cestoda (Tapeworms), e.g. *Diphyllobothrium spp., Tenia spp., Echinococcus spp., Dipylidium caninum, Multiceps spp., Hymenolepis spp., Mesocestoides spp., Vampirolepis spp., Moniezia spp., Anoplocephala spp., Sirometra spp., Anoplocephala spp.,* and *Hymenolepis spp.*

The compounds of formula I and compositions containing them are particularly useful for the control of pests from the orders Diptera, Siphonaptera and Ixodida.

Moreover, the use of the compounds of formula I and compositions containing them for combating mosquitoes is especially preferred.

The use of the compounds of formula I and compositions containing them for combating flies is a further preferred embodiment of the present invention.

Furthermore, the use of the compounds of formula I and compositions containing them for combating fleas is especially preferred.

The use of the compounds of formula I and compositions containing them for combating ticks is a further preferred embodiment of the present invention.

The compounds of formula I also are especially useful for combating endoparasites (roundworms nematoda, thorny headed worms and planarians).

Administration can be carried out both prophylactically and therapeutically.

Administration of the active compounds is carried out directly or in the form of suitable preparations, orally, topically/dermally or parenterally.

For oral administration to warm-blooded animals, the formula I compounds may be formulated as animal feeds, animal feed premixes, animal feed concentrates, pills, solutions, pastes, suspensions, drenches, gels, tablets, boluses and capsules. In addition, the formula I compounds may be administered to the animals in their drinking water. For oral administration, the dosage form chosen should provide the animal with 0.01 mg/kg to 100 mg/kg of animal body weight per day of the formula I compound, preferably with 0.5 mg/kg to 100 mg/kg of animal body weight per day.

Alternatively, the formula I compounds may be administered to animals parenterally, for example, by intraruminal, intramuscular, intravenous or subcutaneous injection. The formula I compounds may be dispersed or dissolved in a physiologically acceptable carrier for subcutaneous injection. Alternatively, the formula I compounds may be formulated into an implant for subcutaneous administration. In addition the formula I compound may be transdermally administered to animals. For parenteral administration, the dosage form chosen should provide the animal with 0.01 mg/kg to 100 mg/kg of animal body weight per day of the formula I compound.

The formula I compounds may also be applied topically to the animals in the form of dips, dusts, powders, collars, medallions, sprays, shampoos, spot-on and pour-on formulations and in ointments or oil-in-water or water-in-oil emulsions. For topical application, dips and sprays usually contain 0.5 ppm to 5,000 ppm and preferably 1 ppm to 3,000 ppm of the formula I compound. In addition, the formula I compounds may be formulated as ear tags for animals, particularly quadrupeds such as cattle and sheep.

Suitable preparations are:
- Solutions such as oral solutions, concentrates for oral administration after dilution, solutions for use on the skin or in body cavities, pouring-on formulations, gels;
- Emulsions and suspensions for oral or dermal administration; semi-solid preparations;
- Formulations in which the active compound is processed in an ointment base or in an oil-in-water or water-in-oil emulsion base;
- Solid preparations such as powders, premixes or concentrates, granules, pellets, tablets, boluses, capsules; aerosols and inhalants, and active compound-containing shaped articles.

Compositions suitable for injection are prepared by dissolving the active ingredient in a suitable solvent and optionally adding further ingredients such as acids, bases, buffer salts, preservatives, and solubilizers. The solutions are filtered and filled sterile.

Suitable solvents are physiologically tolerable solvents such as water, alkanols such as ethanol, butanol, benzyl alcohol, glycerol, propylene glycol, polyethylene glycols, N-methyl-pyrrolidone, 2-pyrrolidone, and mixtures thereof.

The active compounds can optionally be dissolved in physiologically tolerable vegetable or synthetic oils which are suitable for injection.

Suitable solubilizers are solvents which promote the dissolution of the active compound in the main solvent or prevent its precipitation. Examples are polyvinylpyrrolidone, polyvinyl alcohol, polyoxyethylated castor oil, and polyoxyethylated sorbitan ester.

Suitable preservatives are benzyl alcohol, trichlorobutanol, p-hydroxybenzoic acid esters, and n-butanol.

Oral solutions are administered directly. Concentrates are administered orally after prior dilution to the use concentration. Oral solutions and concentrates are prepared according to the state of the art and as described above for injection solutions, sterile procedures not being necessary.

Solutions for use on the skin are trickled on, spread on, rubbed in, sprinkled on or sprayed on.

Solutions for use on the skin are prepared according to the state of the art and according to what is described above for injection solutions, sterile procedures not being necessary.

Further suitable solvents are polypropylene glycol, phenyl ethanol, phenoxy ethanol, ester such as ethyl or butyl acetate, benzyl benzoate, ethers such as alkyleneglycol alkylether, e.g. dipropylenglycol monomethylether, ketons such as acetone, methylethylketone, aromatic hydrocarbons, vegetable and synthetic oils, dimethylformamide, dimethylacetamide, transcutol, solketal, propylencarbonate, and mixtures thereof.

It may be advantageous to add thickeners during preparation. Suitable thickeners are inorganic thickeners such as bentonites, colloidal silicic acid, aluminium monostearate, organic thickeners such as cellulose derivatives, polyvinyl alcohols and their copolymers, acrylates and methacrylates.

Gels are applied to or spread on the skin or introduced into body cavities. Gels are prepared by treating solutions which have been prepared as described in the case of the injection solutions with sufficient thickener that a clear material having an ointment-like consistency results. The thickeners employed are the thickeners given above.

Pour-on formulations are poured or sprayed onto limited areas of the skin, the active compound penetrating the skin and acting systemically.

Pour-on formulations are prepared by dissolving, suspending or emulsifying the active compound in suitable skin-compatible solvents or solvent mixtures. If appropriate, other auxiliaries such as colorants, bioabsorption-promoting substances, antioxidants, light stabilizers, adhesives are added.

Suitable solvents which are: water, alkanols, glycols, polyethylene glycols, polypropylene glycols, glycerol, aromatic alcohols such as benzyl alcohol, phenylethanol, phenoxyethanol, esters such as ethyl acetate, butyl acetate, benzyl benzoate, ethers such as alkylene glycol alkyl ethers such as dipropylene glycol monomethyl ether, diethylene glycol mono-butyl ether, ketones such as acetone, methyl ethyl ketone, cyclic carbonates such as propylene carbonate, ethylene carbonate, aromatic and/or aliphatic hydrocarbons, vegetable or synthetic oils, DMF, dimethylacetamide, n-alkylpyrrolidones such as methylpyrrolidone, n-butylpyrrolidone or n-octylpyrrolidone, N-methylpyrrolidone, 2-pyrrolidone, 2,2-dimethyl-4-oxymethylene-1,3-diox- olane and glycerol formal.

Suitable colorants are all colorants permitted for use on animals and which can be dissolved or suspended.

Suitable absorption-promoting substances are, for example, DMSO, spreading oils such as isopropyl myristate, dipropylene glycol pelargonate, silicone oils and copolymers thereof with polyethers, fatty acid esters, triglycerides, fatty alcohols.

Suitable antioxidants are sulfites or metabisulfites such as potassium metabisulfite, ascorbic acid, butylhydroxytoluene, butylhydroxyanisole, tocopherol.

Suitable light stabilizers are, for example, novantisolic acid.

Suitable adhesives are, for example, cellulose derivatives, starch derivatives, polyacrylates, natural polymers such as alginates, gelatin.

Emulsions can be administered orally, dermally or as injections.

Emulsions are either of the water-in-oil type or of the oil-in-water type.

They are prepared by dissolving the active compound either in the hydrophobic or in the hydrophilic phase and homogenizing this with the solvent of the other phase with the aid of suitable emulsifiers and, if appropriate, other auxiliaries such as colorants, absorption-promoting substances, preservatives, antioxidants, light stabilizers, viscosity-enhancing substances.

Suitable hydrophobic phases (oils) are:
- liquid paraffins, silicone oils, natural vegetable oils such as sesame oil, almond oil, castor oil, synthetic triglycerides such as caprylic/capric biglyceride, triglyceride mixture with vegetable fatty acids of the chain length C₈-C₁₂ or other specially selected natural fatty acids, partial glyceride mixtures of saturated or unsaturated fatty acids possibly also containing hydroxyl groups, mono- and diglycerides of the C₈-C₁₀ fatty acids,
- fatty acid esters such as ethyl stearate, di-n-butyryl adipate, hexyl laurate, dipropylene glycol perlargonate, esters of a branched fatty acid of medium chain length with saturated fatty alcohols of chain length C₁₆-C₁₈, isopropyl myristate, isopropyl palmitate, caprylic/capric acid esters of saturated fatty alcohols of chain length C₁₂-C₁₈, isopropyl stearate, oleyl oleate, decyl oleate, ethyl oleate, ethyl lactate, waxy fatty acid esters such as synthetic duck coccygeal gland fat, dibutyl phthalate, diisopropyl adipate, and ester mixtures related to the latter,
- fatty alcohols such as isotridecyl alcohol, 2-octyldodecanol, cetylstearyl alcohol, oleyl alcohol, and
- fatty acids such as oleic acid and
- mixtures thereof.

Suitable hydrophilic phases are: water, alcohols such as propylene glycol, glycerol, sorbitol and mixtures thereof.

Suitable emulsifiers are:
- non-ionic surfactants, e.g. polyethoxylated castor oil, polyethoxylated sorbitan monooleate, sorbitan monostearate, glycerol monostearate, polyoxyethyl stearate, alkylphenol polyglycol ether;
- ampholytic surfactants such as di-sodium N-lauryl-p-iminodipropionate or lecithin;
- anionic surfactants, such as sodium lauryl sulfate, fatty alcohol ether sulfates, mono/dialkyl polyglycol ether orthophosphoric acid ester monoethanolamine salt;
- cation-active surfactants, such as cetyltrimethylammonium chloride.

Suitable further auxiliaries are: substances which enhance the viscosity and stabilize the emulsion, such as carboxymethylcellulose, methylcellulose and other cellulose and starch derivatives, polyacrylates, alginates, gelatin, gum arabic, polyvinylpyrrolidone, polyvinyl alcohol, copolymers of methyl vinyl ether and maleic anhydride, polyethylene glycols, waxes, colloidal silicic acid or mixtures of the substances mentioned.

Suspensions can be administered orally or topically/dermally. They are prepared by suspending the active compound in a suspending agent, if appropriate with addition of other auxiliaries such as wetting agents, colorants, bioabsorption-promoting substances, preservatives, antioxidants, light stabilizers.

Liquid suspending agents are all homogeneous solvents and solvent mixtures.

Suitable wetting agents (dispersants) are the emulsifiers given above.

Other auxiliaries which may be mentioned are those given above.

Semi-solid preparations can be administered orally or topically/dermally. They differ from the suspensions and emulsions described above only by their higher viscosity.

For the production of solid preparations, the active compound is mixed with suitable excipients, if appropriate with addition of auxiliaries, and brought into the desired form.

Suitable excipients are all physiologically tolerable solid inert substances. Those used are inorganic and organic substances. Inorganic substances are, for example, sodium chloride, carbonates such as calcium carbonate, hydrogencarbonates, aluminium oxides, titanium oxide, silicic acids, argillaceous earths, precipitated or colloidal silica, or phosphates. Organic substances are, for example, sugar, cellulose, foodstuffs and feeds such as milk powder, animal meal, grain meals and shreds, starches.

Suitable auxiliaries are preservatives, antioxidants, and/or colorants which have been mentioned above.

Other suitable auxiliaries are lubricants and glidants such as magnesium stearate, stearic acid, talc, bentonites, disintegration-promoting substances such as starch or crosslinked polyvinylpyrrolidone, binders such as starch, gelatin or linear polyvinylpyrrolidone, and dry binders such as microcrystalline cellulose.

The compositions which can be used in the invention can comprise generally from about 0.001 to 95% of the compound of formula I.

Generally it is favorable to apply the compounds of formula I in total amounts of 0.5 mg/kg to 100 mg/kg per day , preferably 1 mg/kg to 50 mg/kg per day.

Ready-to-use preparations contain the compounds acting against parasites, preferably ectoparasites, in concentrations of 10 ppm to 80 per cent by weight, preferably from 0.1 to 65 per cent by weight, more preferably from 1 to 50 per cent by weight, most preferably from 5 to 40 per cent by weight.

Preparations which are diluted before use contain the compounds acting against ectoparasites in concentrations of 0.5 to 90 per cent by weight, preferably of 1 to 50 per cent by weight.

Furthermore, the preparations comprise the compounds of formula I against endoparasites in concentrations of 10 ppm to 2 per cent by weight, preferably of 0.05 to 0.9 per cent by weight, very particularly preferably of 0.005 to 0.25 per cent by weight.

In a preferred embodiment of the present invention, the compositions comprising the compounds of formula I them are applied dermally / topically.

In a further preferred embodiment, the topical application is conducted in the form of compound-containing shaped articles such as collars, medallions, ear tags, bands for fixing at body parts, and adhesive strips and foils.

Generally it is favorable to apply solid formulations which release compounds of formula I in total amounts of 10 mg/kg to 300 mg/kg, preferably 20 mg/kg to 200 mg/kg, most preferably 25 mg/kg to 160 mg/kg body weight of the treated animal in the course of three weeks.

For the preparation of the shaped articles, thermoplastic and flexible plastics as well as elastomers and thermoplastic elastomers are used. Suitable plastics and elastomers are polyvinyl resins, polyurethane, polyacrylate, epoxy resins, cellulose, cellulose derivatives, polyamides and polyester which are sufficiently compatible with the compounds of formula I. A detailed list of plastics and elastomers as well as preparation procedures for the shaped articles is given e.g. in WO 03/086075.

The active compounds can also be used as a mixture with synergists or with other active compounds which act against pathogenic endo- and ectoparasites.

The following list of pesticides together with which the compounds according to the invention can be used, is intended to illustrate the possible combinations, but not to impose any limitation:
Organophosphates: Acephate, Chlorfenvinphos, Diazinon, Dichlorvos, Dicrotophos, Dimethoate, Ethion, Fenitrothion, Fenthion, Isoxathion, Malathion, Phenthoate, Phosalone, Phosmet, Phoxim, Pirimiphos-methyl, Profenofos, Prothiofos, Sulprophos, Triazophos, Trichlorfon, Quintiofos, Coumaphos, Chlorphoxim, Bromophos-ethyl, 2,3-p-Dioxanedithiol-S,S-bis(O,O-diethylphosphorodithionat);
Carbamates: Alanycarb, Benfuracarb, Carbaryl, Carbosulfan, Fenoxycarb, Furathiocarb, Indoxacarb, Triazamate;
Pyrethroids: alpha-Cypermethrin, Deltamethrin, Ethofenprox, Fenvalerate, Cyhalothrin, Lambda-Cyhalothrin, Permethrin, Silafluofen, Tau-Fluvalinate, Tralomethrin, Zeta-Cypermethrin, Flumethrin, Cyfluthrin and its enantiomers and stereomers, Cypermethrin;
Arthropod growth regulators: a) chitin synthesis inhibitors: benzoylureas: Chlorfluazuron, Cyromazine, Diflubenzuron, Flucycloxuron, Flufenoxuron, Hexaflumuron, Lufenuron, Novaluron, Teflubenzuron, Triflumuron; Buprofezin, Diofenolan, Hexythiazox, Etoxazole, Clofentazine; b) ecdysone antagonists: Halofenozide, Methoxyfenozide, Tebufenozide; c) juvenoids: Pyriproxyfen, Methoprene, Fenoxycarb; d) lipid biosynthesis inhibitors: Spirodiclofen;
Neonicotinoids: Acetamiprid, Clothianidin, Flonicamid, Imidacloprid, Nitenpyram, Thiacloprid, Thiamethoxam;
Synthetic coccidiosis compounds, polyetherantibiotics: Amprolium, Robenidin, Toltrazuril, Monensin, Salinomycin, Maduramicin, Lasalocid, Narasin, Semduramicin;

Various: Abamectin (Avermectins), Acequinocyl, Amitraz, Azadirachtin, Bifenazate, *Bacillus thuringiensis, Bacillus subtilis,* Cartap, Chlorfenapyr, Chlordimeform, Cyromazine, Diafenthiuron, Dinetofuran, Diofenolan, Emamectin, Endosulfan, Epsiprantel, Ethiprole, Fenazaquin, Fipronil, Formetanate, Formetanate hydrochloride, Hydramethylnon, Indoxacarb, 4-{(2Z)-2-({[4-(trifluoro-methoxy)anilino] carbonyl} hydrazono)-2-[3-(trifluoromethyl)-phenyl]ethyl} benzo-nitrile, L-2,3,5,6-tetrahydro-6-phenyl-imidazothiazole, Levamisole, Milbemectin (Milbemycins), Moxidectin, Praziquantel, Pyrantel, Pyridaben, Pymetrozine, Selamectin, Spinosad, Sulfur, Tebufenpyrad, and Thiocyclam.

In general, "parasiticidally effective amount" means the amount of active ingredient needed to achieve an observable effect on growth, including the effects of necrosis, death, retardation, prevention, and removal, destruction, or otherwise diminishing the occurrence and activity of the target organism. The parasiticidally effective amount can vary for the various compounds/compositions used in the invention. A parasiticidally effective amount of the compositions will also vary according to the prevailing conditions such as desired parasiticidal effect and duration, target species, mode of application, and the like.

### Examples of action against parasites

### 1. Screening method to test contact activity against stable fly, yellowfever mosquito, house mosquito, malaria mosquito, cat flea, and brown dog tick via glass contact

Glass vials (20 ml scintillation vials) were treated with 0.5 ml of a solution of active ingredient in acetone. Each vial was rolled uncapped for ca. 10 minutes to allow the a.i. to completely coat the vial and to allow for full drying of the acetone. Insects or ticks were placed into each vial. The vials were kept at 22 °C and were observed for treatment effects at various time intervals. Results are presented in Table I.

### 2. Screening method to test contact activity against yellowfever mosquito, southern house mosquito, and malaria mosquito larvae via water treatment

Well plates were used as test arenas. The active ingredient was dissolved in acetone and diluted with water to obtain the concentrations needed. The final solutions containing appr. 1% acetone were placed into each well. Approximately 10 mosquito larvae (4^{th}-instars) in 1 ml water were added to each well. Larvae were fed one drop of liver powder each day. The dishes were covered and maintained at 22°C. Mortality was recorded daily and dead larvae and live or dead pupae were removed daily. At the end of the test remaining live larvae were recorded and percent mortality was calculated. Results are shown in Table I.

Each test was replicated at least 3 times.

### Results

Tests conducted with compounds of formula I-1 and I-2 showed the following results:

**Table I. Activity against various species.**

| Pest Common Name | Pest Latin Name | Rate | Days or Hours to achieve 100% mortality |
|---|---|---|---|
| Screening method to test contact activity via glass contact | | | |
| stable fly | *Stomoxys calcitrans* | 10 ppm | 4 hours |
| yellowfever mosquito | *Aedes aegypti* | 10 ppm | 4 hours |
| house mosquito | *Culex quinquefasciatus* | 0.5 ppm | 4 hours |
| malaria mosquito | *Anopheles albimanus* | 1 ppm | 1 day |
| cat flea | *Ctenocephalides felis* | 100 ppm | 2 days |
| brown dog tick | *Rhipicephalus sanguineus* | 10 ppm | 3-5 days |
| Screening method to test contact activity via water treatment | | | |
| yellowfever mosquito | *Aedes aegypti* | 10 ppm | 2 days |
| house mosquito | *Culex quinquefasciatus* | 10 ppm | 1 day |
| malaria mosquito | *Anopheles albimanus* | 1.0 ppm | 1 day |

### 3. Activity against cat flea in an "articifial dog" apparatus

The active ingredient was dissolved in acetone and mixed with an appropriate volume of defibrinated cattle blood. 5 ml of treated blood were poured into a feeding chamber fitted with a paraffin wax film membrane. The chamber with the treated blood was placed over a flea feeding chamber. The test was repeated for each of 5 dose concentrations of each of the active ingredients. Treatment effects, including knockdown, failure to feed after 24 hours, failure to lay eggs, etc, were observed at various intervals. Control tests were conducted with acetone / blood mixtures.

### Results

Tests conducted with 100 ppm of compounds of formula I-1 and I-2 showed an over 60% killing rate of *Ctenocephalides felis.*

## Claims

1. The non-therapeutic use of compounds of formula I 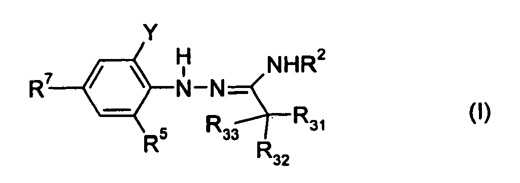 wherein
R⁷ is chlorine or trifluoromethyl;
R⁵ and Y are each independently chlorine or bromine;
R² is C₁-C₆-alkyl, C₃-C₆-alkenyl, C₃-C₆-alkynyl, or
C₃-C₆-cycloalkyl which may be substituted with 1 to 3 halogen atoms, or
C₂-C₄-alkyl which is substituted by C₁-C₄-alkoxy;
R³¹ and R³² are C₁-C₆-alkyl or may be taken together to form C₃-C₆-cycloalkyl which may be unsubstituted or substituted by 1 to 3 halogen atoms;
R³³ is hydrogen or C₁-C₆-alkyl,
or the enantiomers or veterinarily acceptable salts thereof
for combating parasites in and on animals.

2. The use according to claim 1 wherein the compound of formula I is a compound of formula I-1.

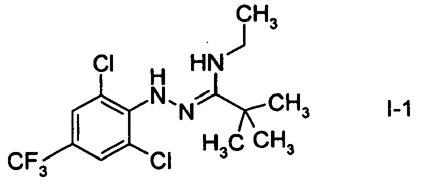

3. The use according to claim 1 wherein the compound of formula I is a compound of formula I-2.

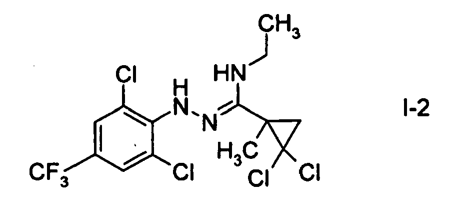

4. The use as claimed in claims 1 to 3 wherein the parasites are selected from the Diptera, Siphonaptera, and Ixodida orders.

5. The use as claimed in claims 1 to 4 wherein the animals are cats or dogs.

6. A non-therapeutic method for treating, controlling, preventing or protecting animals against infestation or infection by parasites which comprises orally, topically or parenterally administering or applying to the animals a parasiticidally effective amount of a compound of formula I as defined in any one of claims 1 to 3.

7. The method as claimed in claim 6 wherein the parasites are selected from the Diptera, Siphonaptera, and Ixodida orders.

8. The method as claimed in claims 6 or 7 wherein the animals are cats or dogs.

9. A process for the preparation of a composition for treating, controlling, preventing or protecting animals against infestation or infection by parasites which comprises combining a parasiticidally effective amount of a compound of formula I as defined in any one of claims 1 to 3 with an acceptable carrier.

## Patentansprüche

1. Nichttherapeutische Verwendung von Verbindungen der Formel I 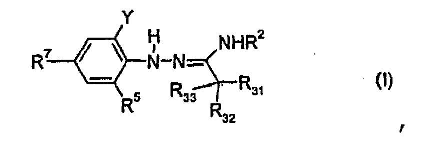 worin
R⁷ Chlor oder Trifluormethyl bedeutet;
R⁵ und Y jeweils unabhängig Chlor oder Brom bedeuten;
R² C₁-C₆-Alkyl, C₃-C₆-Alkenyl, C₃-C₆-Alkinyl bedeutet, oder C₃-C₆-Cycloalkyl, das mit 1 bis 3 Halogenatomen substituiert sein kann, oder C₂-C₄-Alkyl, das durch C₁-C₄-Alkoxy substituiert sein kann, bedeutet;
R³¹ und R³² C₁-C₆-Alkyl bedeuten oder gemeinsam C₃-C₆-Cycloalkyl, das unsubstituiert oder durch 1 bis 3 Halogenatome substituiert sein kann, bilden können;
R³³ Wasserstoff oder C₁-C₆-Alkyl bedeutet,
oder deren Enantiomeren oder tierärztlich unbedenklichen Salzen
für die Bekämpfung von Parasiten in und an Tieren.

2. Verwendung nach Anspruch 1, wobei es sich bei der Verbindung der Formel I um eine Verbindung der Formel I-1 handelt

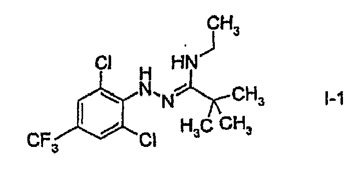

3. Verwendung nach Anspruch 1, wobei es sich bei der Verbindung der Formel I um eine Verbindung der Formel I-2 handelt

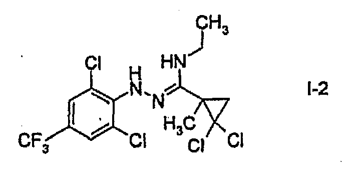

4. Verwendung nach den Ansprüchen 1 bis 3, wobei die Parasiten aus den Ordnungen Diptera, Siphonaptera und Ixodida stammen.

5. Verwendung nach den Ansprüchen 1 bis 4, wobei es sich bei den Tieren um Katzen oder Hunde handelt.

6. Nichttherapeutisches Verfahren zur Behandlung, zur Bekämpfung, zur Vorbeugung oder zum Schützen von Tieren gegen Befall oder Infektion durch Parasiten, **dadurch gekennzeichnet, daß** man den Tieren eine parasitizidwirksame Menge einer Verbindung der Formel I gemäß der Definition nach einem der Ansprüche 1 bis 3 oral, topisch oder parenteral verabreicht.

7. Verfahren nach Anspruch 6, wobei die Parasiten aus den Ordnungen Diptera, Siphonaptera und Ixodida stammen.

8. Verfahren nach Anspruch 6 oder 7, wobei es sich bei den Tieren um Katzen oder Hunde handelt.

9. Verfahren zur Herstellung einer Zusammensetzung für die Behandlung, die Bekämpfung, die Vorbeugung oder den Schutz von Tieren gegen Befall oder Infektion durch Parasiten, bei dem man eine parasitizidwirksame Menge einer Verbindung der Formel I gemäß der Definition nach einem der Ansprüche 1 bis 3 mit einem unbedenklichen Träger kombiniert.

## Revendications

1. Utilisation non thérapeutique de composés de formule I 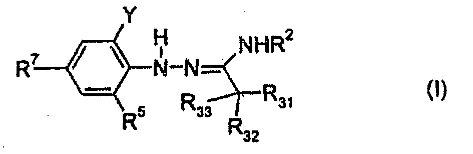 dans laquelle
R⁷ est un atome de chlore ou un groupe trifluorométhyle ;
R⁵ et Y sont, chacun indépendamment, un atome de chlore ou un atome de brome ;
R² est un groupe alkyle en C₁-C₆, un groupe alcényle en C₃-C₆, un groupe alcynyle en C₃-C₆, ou un groupe cycloalkyle en C₃-C₆ qui peut être substitué par 1 à 3 atomes d'halogène, ou un groupe alkyle en C₂-C₄ qui est substitué par un groupe alcoxy en C₁-C₄ ;
R³¹ et R³² sont un groupe alkyle en C₁-C₆ ou peuvent être pris conjointement pour former un groupe cycloalkyle en C₃-C₆ qui peut être non substitué ou substitué par 1 à 3 atomes d'halogène ;
R³³ est un atome d'hydrogène ou un groupe alkyle en C₁-C₆ ;
ou des énantiomères ou des sels acceptables sur le plan vétérinaire de ceux-ci
pour la lutte contre des parasites chez et sur des animaux.

2. Utilisation selon la revendication 1, dans laquelle le composé de formule I est un composé de formule I-1.

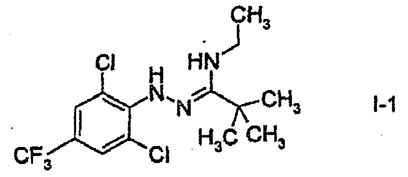

3. Utilisation selon la revendication 1, dans laquelle le composé de formule I est un composé de formule I-2.

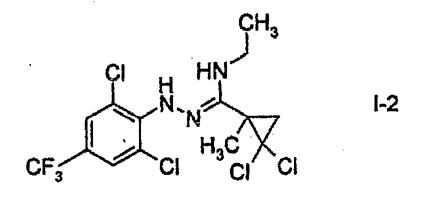

4. Utilisation selon les revendications 1 à 3, dans laquelle les parasites sont choisis parmi les ordres Diptera, Siphonaptera et Ixodida.

5. Utilisation selon les revendications 1 à 4, dans laquelle les animaux sont des chats ou des chiens.

6. Méthode non thérapeutique de traitement, de maîtrise, de prévention ou de protection d'animaux contre une infestation ou une infection par des parasites, qui comprend l'administration par voie orale, locale ou parentérale, ou l'application sur les animaux, d'une quantité efficace du point de vue parasiticide d'un composé de formule I selon l'une quelconque des revendications 1 à 3.

7. Méthode selon la revendication 6, dans laquelle les parasites sont choisis parmi les ordres Diptera, Siphonaptera et Ixodida.

8. Méthode selon les revendications 6 ou 7, dans laquelle les animaux sont des chats ou des chiens.

9. Procédé de préparation d'une composition pour le traitement, la maîtrise, la prévention ou la protection d'animaux contre une infestation ou une infection par des parasites, qui comprend la combinaison d'une quantité efficace du point de vue parasiticide d'un composé de formule I selon l'une quelconque des revendications 1 à 3 avec un support acceptable.
